# EUROPEAN PATENT APPLICATION

(11) **EP 2 727 625 A1**
(43) Date of publication of application: **07.05.2014**
(21) Application number: 13005104.8
(22) Date of filing: 25.10.2013
(51) Int. Cl.: A61N 2/00, A61N 2/06

(54) **Device having a magnetic insert**

(30) Priority: 30.10.2012 HU 1200211
(71) Applicant: Tokar, Ferencne, 5700 Gyula (HU)
(72) Inventor: Tokar, Ferencne, 5700 Gyula (HU)
(74) Representative: Emri, Jozsefné

(57) **Abstract**

The invention relates to a device having a magnetic insert that is easy, simple to use by anyone for improving the physical well-being and comfort of people, for enhancing their physical and mental performance, for relieving stress, and for increasing the energy levels of living organisms, for harmonizing their physical and energy bodies, different points, areas thereof, which can also be used in the field of beauty care and sports.

The main parts of the device having a magnetic insert are the holder element, the permanent magnetic insert and the cap with a cover plate. The device having a magnetic insert can be expediently fitted into a hand-held holder unit.

## Description

The invention relates to a device having a magnetic insert for improving the physical well-being and comfort of people, for enhancing their physical and mental performance, for relieving stress, and for increasing the energy levels of living organisms, for harmonizing their physical and energy bodies, different points, areas thereof, which can also be used in the field of beauty care and sports.

Magnetic biostimulation is scientifically proven to play an important role in health promotion. The bioelectric activity of the cells of living organisms is measurable (ECG, EEG). It is a fact that due to the declining magnetism of the Earth the cellular metabolism of living organisms may slow down. By replenishing magnetism deficiency, the energy level (ATP level) of cells can be increased, the self-regulating capacity of the organism can be supported.

Various types of magnetic devices, magnetic equipment are commercially available. E.g. Magnapress and Minat Magnetic include a permanent magnet, B.P.R.S is an electromagnetic device; and e.g. the BREMER, Magneter and Promag devices use the pulsed magnetic method.

Patent No. HU224 241 relates to a magnetic appliance comprising a hand-held sized, tube-shaped housing, and at one end a contact head containing a permanent magnet, with a contact surface for contact with the skin.

The permanent magnet is made up of at least a first piece and a second piece in such a way that the first piece includes the whole contact surface, towards which the north pole of the first piece faces, and the second piece is mounted on the side of the first piece opposite to the contact surface in such a way that the second piece has opposite polarity to the first piece.

The know solutions often have a strong permanent magnet, their use is recommended with complex point combinations, thus the magnetization process is often very complicated, cumbersome for lay people, it may not be done alone, assistance may be needed, and the magnet in the appliances may cause irritation to sensitive users, furthermore due to the price of the appliances, they are unaffordable for families of average financial means.

The aim of the invention is to create a magnetic device that is of simple design, easy to use, portable, not causing irritation to the users, pleasant to the touch, with a magnetic field strength matching their lifestyles, energy requirements. A further aim is to make the device easy, practical, simple, comfortable to use for lay users, even without assistance, and to make it affordable for families of average financial means, by providing good value for money.

The present invention relates to a device having a magnetic insert for improving the physical well-being and comfort of people, for enhancing their physical and mental performance, for relieving stress, and for increasing the energy levels of living organisms, for harmonizing their physical and energy bodies, different points, areas thereof, which can also be used in the field of beauty care and sports, consisting of a holder element, a permanent magnetic insert and a cap with a cover plate. The holder element comprises of a handle and a fixing unit. The magnetic insert mounted on the upper part of the holder element, on the fixing unit, is fixed firmly to the holder element by the cap. The handle of the holder element can be rod- or disk-shaped, the magnetic insert is disk-shaped, the cap can be closed or open. The fixing unit of the holder element and the inner side of the cap are threaded.

The magnetic insert can be gold plated, and the mineral Tourmaline can be fixed on the holder element.

The device having a magnetic insert can be equipped with a massage cap as well.

Two or more devices having a magnetic insert can preferably be fitted into a hand-held holder unit.

The invention is described in detail below with reference to the following figures, where
- Figure 1: shows a longitudinal sectional view of the device having a magnetic insert, comprising a rod-shaped handle and a closed cap;
- Figure 2: shows a longitudinal sectional view of the device having a magnetic insert, comprising a rod-shaped handle and an open cap;
- Figure 3: shows a longitudinal sectional view of the device having a magnetic insert, comprising a disk-shaped handle and a closed cap;
- Figure 4: shows a longitudinal sectional view of the device having a magnetic insert, comprising a disk-shaped handle and an open cap;
- Figure 5: shows a longitudinal sectional view of the massage cap;
- Figure 6: shows a side view of the hand-held holder unit;
- Figure 7: shows a top view of the lower plate of the hand-held holder unit.

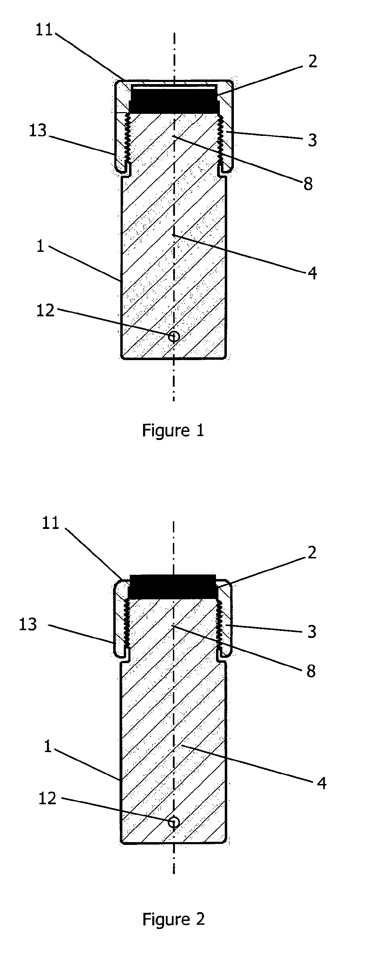

Figure 1 shows the main parts of the invention: the holder element 1, the magnetic insert 2, and the cap 3.

The holder element 1 comprises a handle 4 portion and a fixing unit 8. Both the handle 4 and the fixing unit 8 have a circular cross-section, and the fixing unit 8 is threaded. The holder element 1 is delimited at the top of the fixing unit 8 by a closing surface. The closing surface is a flat surface. The disk-shaped magnetic insert 2 is mounted on the closing surface of the holder element 1.

The cap 3 has a cup-like design, its side portion is a cylindrical cap mantle 13, the inner part of which is threaded. The upper, horizontal portion of the cap 3 is a cover plate 11. The disk-shaped magnetic insert 2 mounted on the closing surface of the holder element 1 is fixed to the holder element 1 by the cap 3. In the shown embodiment the cover plate 11 is closed, the upper part of the magnetic insert 2 is covered: the cover plate 11 covers the magnetic insert 2. The handle 4 has a bore 12.

In Figure 2 the holder element 1 comprises a rod-shaped handle 4 and a threaded fixing unit 8, and the disk-shaped magnetic insert 2 is mounted on the closing surface of the holder element 1. The cap mantle 13 of the cap 3 is threaded on the inside, its cover plate 11 is open: the cover plate 11 has an opening. The shape of the opening matches the shape of the magnetic insert 2. The magnetic insert 2 mounted on the closing surface of the holder element 1 is fixed to the holder element 1 by the open cap 3. In the shown embodiment the magnetic insert 2 protrudes slightly from the plane of the cover plate 11.

The handle 4 has a bore 12.

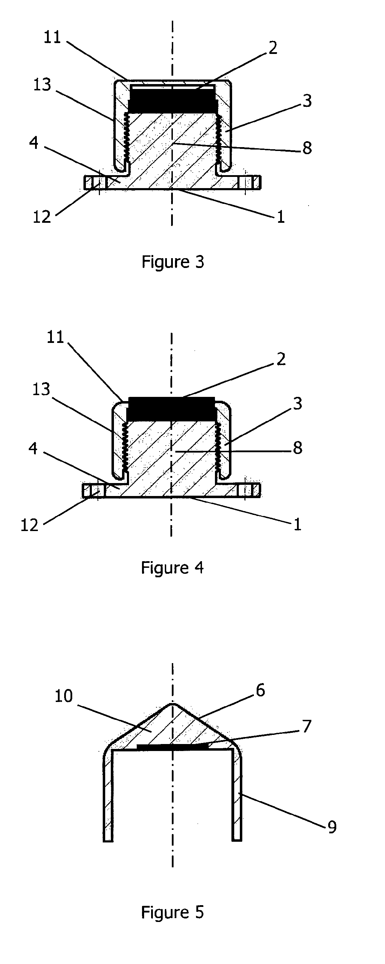

In Figure 3 the handle 4 of the holder element 1 is disk-shaped, the fixing unit 8 is threaded. The cover plate 11 of the cap 3 fixing the magnetic insert 2 mounted on the holder element 1 is closed, its cap mantle 13 is threaded. In the shown embodiment the cover plate 11 covers the whole upper part of the magnetic insert 2.

The handle 4 has two bores 12.

The holder element 1 of the embodiment shown in Figure 4 comprises a disk-shaped handle 4 and a threaded fixing unit 8. The magnetic insert 2 is mounted thereon, and fixed to the holder element 1 by an open cap 3. The cover plate 11 of the cap 3 has an opening, the inner side of the cap mantle 13 is threaded. The magnetic insert 2 protrudes slightly from the plane of the cover plate 11.

The handle 4 has two bores 12.

Figure 5 shows the massage cap 6 comprising a cone-shaped massage surface 10 and a mantle 9.

An iron plate 7 is fixed to the lower part of the cone-shaped massage surface 10.

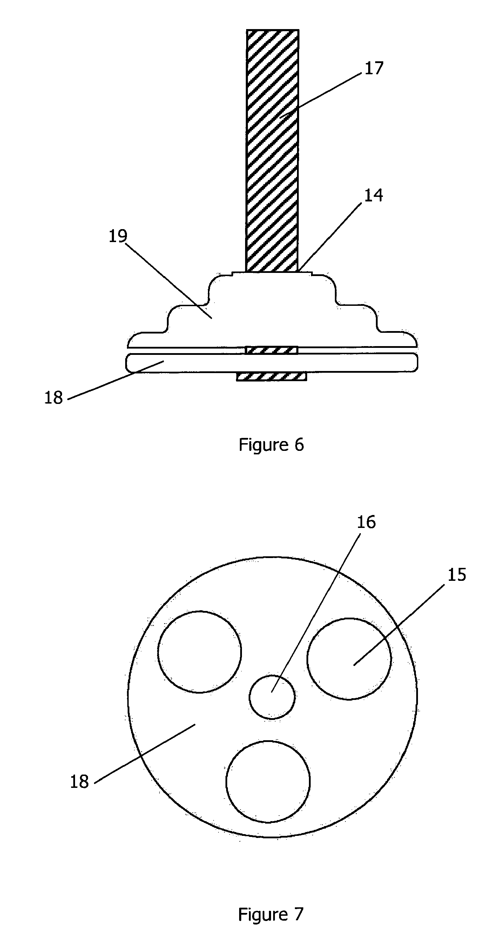

Figure 6 shows a circular hand-held holder unit 14 comprising: a grip element 17, a lower plate 18 and an upper plate 19. The lower plate 18 and the upper plate 19 are mountable on the grip element 17.

The lower plate 18 is fixed firmly by the lower flared-end of the grip element 17.

The part of the arched upper plate 19 connecting to the grip element 17 is threaded, thus the distance between the lower plate 18 and the upper plate 19 is adjustable.

Figure 7 shows the circular lower plate 18 with three device holding slots 15 formed in it. The device holding slots 15 are formed around the grip element slot 16. In the shown embodiment three devices having a magnetic insert can be fitted into the hand-held holder unit 14.

The main parts of the device according to the invention are: the holder element 1, the magnetic insert 2, and the cap 3.

The lower portion of the holder element 1 is the handle 4, its upper portion is the fixing unit 8 delimited at the top by a closing surface. The handle 4 can be rod-shaped or disk-shaped, and preferably has a bore 12. The fixing unit 8 is threaded.

The magnetic insert 2 is preferably disk-shaped and is mounted on the top, closing surface of the holder element 1.

The magnetic insert 2 is a permanent magnet, preferably NeFeB.

Preferably the lower side of the magnetic insert 2 mounted on the closing surface of the holder element 1 is of south polarity, its upper side is of north polarity. Then the upper, north pole side is directed towards the user.

The side portion of the cap 3 is a cylindrical cap mantle 13, the inner part of which is threaded; its upper, horizontal portion is a cover plate 11. The cap 3 is closed if the cover plate 11 is closed, and open if the cover plate 11 has an opening. The shape of the opening preferably matches the shape of the magnetic insert 2.

The cap 3 and the fixing unit 8 are threaded.

The magnetic insert 2 mounted on the top, closing surface of the holder element 1, is fixed to the holder element 1 by an open or closed cap 3 in such a way that the cap 3 is screwed on the fixing unit 8.

Where appropriate, the fixation can be strengthened by gluing.

With a closed cap 3 the cover plate 11 covers the whole upper, north pole side of the magnetic insert 2.

With an open cap 3 the magnetic insert 2 protrudes slightly from the plane of the cover plate 11.

The device having a magnetic insert according to the invention, comprising either a rod-shaped or a disk-shaped handle 4, with either a closed or an open cap 3, can be equipped with a massage cap 6. The massage cap 6 has a cylindrical mantle 9 and a cone-shaped massage surface 10. An iron plate 7 is fixed to the lower part of the massage surface 10. The iron plate 7 can be replaced with an iron bolt.

The circumference of the mantle 9 of the massage cap 6 is larger than the circumference of the cap 3 by just so much that by mounting the massage cap 6 on the cap 3, it is fixed firmly thereon.

The holder element 1, the cap 3 and the massage cap 6 of the device having a magnetic insert according to the invention are made preferably of wood, or plastic.

The size of the device having a magnetic insert is small, it fits into the pocket, hand. Users can wear it preferably around the neck, e.g. on a chain, cord or leather strap passed through the bore 12 of the holder element 1.

The hand-held holder unit 14 is preferably circular or triangular, but it can be rectangular or of any polygonal shape. The hand-held holder unit 14 can hold preferably three, but also more than three devices having a magnetic insert. The size of the device holding slots 15 is such that the device having a magnetic insert, or the holder element 1 thereof fits well into the hand-held holder unit 14.

The upper plate 19 is mountable on the grip element 17 preferably by means of a threaded connection, or where appropriate, by means of a flexible mounting ring (silicone, rubber) slipped on the grip element 17.

The devices having a magnetic insert fitted into the device holding slots 15 of the lower plate 18 are fixed in the hand-held holder unit 14 by the upper plate 19.

The hand-held holder unit 14 is made preferably of wood, or plastic.

According to a preferred embodiment the magnetic device comprises a rod-shaped handle 4, a magnetic insert 2 with the north pole side facing towards the user, and an open or closed cap 3.

According to a preferred embodiment the magnetic device comprises a rod-shaped handle 4, a magnetic insert 2 with the south pole side facing towards the user, and a closed cap 3.

According to a preferred embodiment the magnetic device comprises a disk-shaped handle 4, a magnetic insert 2 with the north pole side facing towards the user, and an open or closed cap 3.

According to a preferred embodiment the magnetic device comprises a disk-shaped handle 4, a magnetic insert 2 with the south pole side facing towards the user, and a closed cap 3.

According to a further preferred embodiment the magnetic insert 2 of the device having a magnetic insert is gold plated.

According to a further preferred embodiment the device having a magnetic insert is equipped with a massage cap 6.

According to a further preferred embodiment the mineral Tourmaline is fixed in the bore 12 of the handle 4 of the device having a magnetic insert.

When using the device having a magnetic insert, the user picks up the device having a magnetic insert, and by holding the handle directs the magnetic insert 2 towards the body part to be treated. The user shall use the device according to the invention by observing the instructions for use concerning, among others, the duration of treatment, the method of treatment, and the desired magnetic field strength.

A device having a magnetic insert can create fields of different strength depending on whether it has an open or a closed cap 3. The use of a closed cap 3 produces a weaker field strength, and the thicker the cover plate 11, the weaker the field strength. A solution with an open cap 3 produces a stronger field strength.

The effect of the device having a magnetic insert can be further enhanced if the magnetic insert 2 fixed by an open or closed cap 3 is gold plated.

The effect can also be enhanced by fixing the mineral Tourmaline on the holder element 1, because when it is used together with the mineral Tourmaline, the mineral transfers infrared waves and anions to the user.

The device having a magnetic insert can be equipped with a massage cap 6. The beneficial effect can be further enhanced by placing the massage surface 10 of the massage cap 6 on the body surface to be treated and making massage movements, because the magnetic waves passing through the massage cap 6 increase the efficiency of manual massage, as they increase the energy level of cells by replenishing magnetism deficiency. The iron plate 7 serves to keep the massage cap 6 firmly on the device according to the invention.

The user can use more than one, preferably two or three devices having a magnetic insert simultaneously.

A device family consists preferably of three devices having a magnetic insert, with two north poles and a south pole, or three north poles directed towards the surface to be treated.

When two devices having a magnetic insert are used, by varying the members of the device family (whether two north poles, or a north and a south pole are directed towards the surface to be treated), four kinds of magnetic field strength can be created with the simultaneous use of the devices having a magnetic insert, by directing the devices having a magnetic insert preferably symmetrically towards the right and left side of the body, the front and back of the body, or the paired body parts, facing each other. The devices having a magnetic insert can create fields of different strength in the following combinations:
a) Two devices having a magnetic insert, each with a closed cap 3 and with the north pole directed towards the body part to be magnetized;
b) Two devices having a magnetic insert, each with an open cap 3 and with the north pole directed towards the body part to be magnetized;
c) Two devices having a magnetic insert, one with a closed cap 3 and with the north pole, and the other with a closed cap 3 and the south pole directed towards the body part to be magnetized;
d) Two devices having a magnetic insert, one with an open cap 3 and with the north pole, and the other with a closed cap 3 and the south pole directed towards the body part to be magnetized.

When more than one devices having a magnetic insert are used, it is expedient to use a hand-held holder unit 14. The devices having a magnetic insert, preferably devices having a magnetic insert comprising a disk-shaped handle 4, are fitted into the device holding slots 15 of the lower plate 18 of the hand-held holder unit 14 in such a way that the handles 4 of the devices having a magnetic insert are between the lower plate 18 and the upper plate 19, and are fixed firmly by screwing on the upper plate 19. It is simple, convenient, efficient to use by holding the grip element 17.

A further advantage of the invention is that by combining it with additional elements, such as the mineral Tourmaline, a massage cap, or using two or more devices, magnetic fields of different strength can be created, and thereby the device having a magnetic insert can be used more efficiently for all age groups.

### List of reference numbers

- 1: holder element
- 2: magnetic insert
- 3: cap
- 4: handle
- 6: massage cap
- 7: iron plate
- 8: fixing unit
- 9: mantle
- 10: massage surface
- 11: cover plate
- 12: bore
- 13: cap mantle
- 14: hand-held holder unit
- 15: device holding slot
- 16: grip element slot
- 17: grip element
- 18: lower plate
- 19: upper plate

## Claims

1. A device having a magnetic insert consisting of a holder element (1), a permanent magnetic insert (2) and a cap (3) with a cover plate (11), **characterized in that** the holder element (1) comprises a handle (4) and a fixing unit (8); the magnetic insert (2) mounted on the upper part of the holder element (1) is fixed firmly to the holder element (1) by the cap (3).

2. The device according to claim 1, **characterized in that** the cover plate (11) of the cap (3) is closed.

3. The device according to claim 1, **characterized in that** the cover plate (11) of the cap (3) is open.

4. The device according to claim 1, **characterized in that** the fixing unit (8) and the cap (3) are threaded.

5. The device according to claim 1, **characterized in that** the handle (4) of the holder element (1) is rod- or disk-shaped.

6. The device according to claim 1, **characterized in that** the holder element (1) has a bore (12).

7. The device according to claim 1, **characterized in that** the mineral Tourmaline is fixed on the holder element (1) in a releasable manner.

8. The device according to claim 1, **characterized in that** the magnetic insert (2) is gold plated.

9. The device according to claim 1, **characterized in that** the holder element (1) and the cap (3) are made of wood or plastic.

10. The device according to any of claims 1-9, **characterized in that** the holder element (1) is equipped with a massage cap (6).

11. The device according to claim 10, **characterized in that** the massage cap (6) comprises an iron plate (7).

12. The device according to claim 1, **characterized in that** the holder element (1) is mountable into a hand-held holder unit (14).
